(19) Europäisches Patentamt
European Patent Office
Office européen des brevets

(11) **EP 4 451 287 A1**

(12) **EUROPEAN PATENT APPLICATION**

(43) Date of publication:
**23.10.2024 Bulletin 2024/43**

(21) Application number: **23305610.0**

(22) Date of filing: **19.04.2023**

(51) International Patent Classification (IPC):
**G16H 50/50** (2018.01)     **G06N 3/045** (2023.01)

(52) Cooperative Patent Classification (CPC):
**G16H 50/50; G06N 3/045; G06N 3/09**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Dassault Systèmes**
**78140 Vélizy-Villacoublay (FR)**

(72) Inventors:
• **BENMAHDI, Meryem**
**78140 Velizy-Villacoublay (FR)**
• **HERMANN, Everton**
**78140 Velizy-Villacoublay (FR)**
• **LIBES, Hugo**
**91120 Palaiseau (FR)**

(74) Representative: **Bandpay & Greuter**
**11 rue Christophe Colomb**
**75008 Paris (FR)**

(54) **PREDICTING VASCULAR BEHAVIOR USING A VASCULAR TWIN**

(57) The disclosure notably relates to a computer-implemented method for predicting vascular behavior of a patient, in particular a hemodynamic behavior. The method comprises providing a vascular model of a circulatory system, one or more measurements of the vascular behavior of the patient, and a surrogate model comprising an artificial neural network. The vascular model represents a general vascular behavior and comprises a plurality of physiological parameters. The surrogate model is configured to predict a simulation of a vascular behavior from the physiological parameters. The method further comprises calibrating the vascular model using the surrogate model and based on the one or more measurements; and predicting the vascular behavior of the patient using the calibrated vascular model.

providing a vascular model of a human circulatory system, the circulatory system comprising a plurality of vessels forming a vascular graph, the vascular model comprising a plurality of physiological parameters, the vascular model representing a general vascular behavior, the vascular model comprising a plurality of submodels each comprising a subset of the plurality of physiological parameters, each submodel of the plurality being respective to a vessel of a plurality of vessels forming a vascular graph; one or more measurements of the vascular behavior of the patient; and a surrogate model comprising an artificial neural networks the surrogate model being configured to predict a simulation of a vascular behavior from the physiological parameters — S10

calibrating the vascular model using the surrogate model and based on the one or more measurements — S20

predicting the vascular behavior of the patient using the calibrated vascular model — S30

FIG. 1

**Description**

**TECHNICAL FIELD**

The disclosure relates to the field of computer programs and systems, and more specifically to a method, system and program for predicting vascular behavior of a patient, in particular a hemodynamic behavior.

**BACKGROUND**

A number of systems and programs are offered on the market of healthcare industry to support healthcare professionals in the diagnosis, interventional planning, and/or follow-up of patients. Such offered systems and programs - which may be also known as healthcare software solutions - are developed for simulation and medical training, research, diagnosis, database storage and/or equipment planning. Virtual or equivalently digital twins are among such offered systems and programs. Virtual twins provide a virtual representations (i.e., the "digital twin") of patients ("physical twin"). Such virtual twins are generated from patient data, and real-time updates on patient and environmental variables based on a set of physics-based simulations. Virtual twins seek to match clinical measurements of the patient's anatomical and physiological features to provide a personalized and reliable model for each patient.

Some solutions based on virtual twins have been provided in the market according to the following documents:

[1] Boileau, Etienne, Perumal Nithiarasu, Pablo J. Blanco, Lucas O. Müller, Fredrik Eikeland Fossan, Leif Rune Hellevik, Wouter P. Donders, Wouter Huberts, Marie Willemet, and Jordi Alastruey. "A Benchmark Study of Numerical Schemes for One-Dimensional Arterial Blood Flow Modelling." International Journal for Numerical Methods in Biomedical Engineering 31, no. 10 (2015): e02732;

[2] Shi, Yubing, Patricia Lawford, and Rodney Hose. "Review of Zero-D and 1-D Models of Blood Flow in the Cardiovascular System." BioMedical Engineering OnLine 10, no. 1 (April 26, 2011): 33;

[3] Lal, Rajnesh. "Data Assimilation and Uncertainty Quantification in Cardiovascular Biomechanics." PhD thesis, Université Montpellier, 2017.

[4] Lombardi, D. "Reduced order modelling for direct and inverse problems in hemodynamics." ROMs for the Biomechanics of Living Organs, hal-03783921;

[5] Tezzele, Marco, Francesco Ballarin, and Gianluigi Rozza. "Combined Parameter and Model Reduction of Cardiovascular Problems by Means of Active Subspaces and POD-Galerkin Methods," 16:185-207, 2018;

[6] Köppl, Tobias, Gabriele Santin, Bernard Haasdonk, and Rainer Helmig. "Numerical Modelling of a Peripheral Arterial Stenosis Using Dimensionally Reduced Models and Kernel Methods." International Journal for Numerical Methods in Biomedical Engineering 34, no. 8 (August 2018);

[7] Fresca, Stefania, and Andrea Manzoni. "Real-Time Simulation of Parameter-Dependent Fluid Flows through Deep Learning-Based Reduced Order Models." Fluids 6, no. 7 (July 18, 2021): 259;

[8] Saito, M., Ikenaga, Y., Matsukawa, M., Watanabe, Y., Asada, T., & Lagree, P.Y. (2011). One-dimensional model for propagation of a pressure wave in a model of the human arterial network: comparison of theoretical and experimental results. Journal of Biomechanical Engineering; and

[9] Lombardi, D. "Inverse Problems in 1D Hemodynamics on Systemic Networks: A Sequential Approach: Unscented Kalman Filter Estimation of Network Parameters." International Journal for Numerical Methods in Biomedical Engineering 30, no. 2 (February 2014): 160-79.

[10] Alessandro Melis. "Gaussian process emulators for 1D vascular models". PhD thesis. University of Sheffield, 2017.

These solutions either require extremely costly simulation in terms of computer resources (e.g., CPU cycles, memory, etc.) or cannot be efficiently calibrated to the anatomical and physiological features of the patient so that the outputs of corresponding virtual twins do not reliably match the patient's clinical measurements.

Within this context, there is still a need for an improved for predicting vascular behavior of a patient, in particular a hemodynamic behavior.

**SUMMARY**

It is therefore provided computer-implemented method for predicting vascular behavior of a patient, in particular a hemodynamic behavior. The method comprising providing a vascular model of a circulatory system, the circulatory system comprising a plurality of vessels forming a vascular graph, the vascular model comprising a plurality of physiological parameters, the vascular model representing a general vascular behavior, the vascular model comprising a plurality of submodels each comprising a subset of the plurality of physiological parameters, each submodel of the plurality being respective to a vessel of a plurality of vessels. The method further comprises providing one or more measurements of the vascular behavior of the patient, and a surrogate model comprising an artificial neural network. The surrogate model is configured to predict a simulation of a vascular behavior from the physiological parameters. The method further comprises calibrating the vascular model using the surrogate model and based on the one or more measurements, and predicting the vascular behavior of the patient using the calibrated vascular model.

The method may comprise one or more of the following:

- the surrogate model comprises a plurality of artificial neural networks, each artificial neural network of the plurality being respective to a vessel of a plurality of vessels, the artificial neural networks of the plurality being connected to each other following the vascular graph;
- the calibrating of the vascular model comprises providing an inverse artificial neural network trained to predict the physiological parameters of the patient given one or more measurements of the vascular behavior of the patient; and determining a value of the plurality of physiological parameters using the provided inverse artificial neural network;
- the method, prior to the providing of the inverse artificial neural network, comprises training the inverse artificial neural network; the training comprising providing a dataset of patients, each entry in said database comprising the plurality of physiological parameters being in correspondence to a simulation result of a patient vascular behavior, the simulation result being computed with the physiological parameters, the physiological parameters reflecting the physiological features; and training the inverse artificial neural network based on the dataset;
- the inverse artificial neural network comprises a first part configured to accept as input a subset of the one or more measurements of the vascular behavior of the patient and to output a reconstruction of the one or more measurements of the vascular behavior of the patient, and a second part configured to accept as input the reconstruction of the one or more measurements of the vascular behavior of the patient, and to output the physiological parameters; wherein the second part optionally comprises one artificial neural network, or a plurality of artificial neural networks, being connected to each other following the vascular graph;
- the training of the inverse artificial neural network based on the dataset comprises minimizing a first discrepancy between the one or more measurement of the vascular behavior of the patient, and a prediction of a vascular behavior simulation using the surrogate model and based on the predicted physiological parameters by the provided inverse artificial neural network;
- the training of the inverse artificial neural network based on the dataset comprises minimizing a second discrepancy between the physiological parameters reflecting the physiological features, and the determined physiological parameters by the provided inverse artificial neural network;
- the physiological parameters comprise at least one or more of mechanical properties of the plurality of vessels, and/or geometrical properties of the plurality of vessels; wherein, optionally, the one or more measurements comprises one or more pressure values and/or one or more flow rate values and/or one or more cross-sectional area values of the plurality of vessels;
- the predicting of the vascular behavior using the calibrated vascular model comprises performing a simulation by the calibrated vascular model using the plurality of models, or predicting a simulation by the calibrated vascular model using the surrogate model;
- further comprising, prior to the providing of a surrogate model, training the surrogate model, the method comprising providing a training dataset, each entry in the training dataset comprises a value for the physiological parameters, and a value for a simulation by the vascular model using the plurality of models, and training the surrogate model using the training dataset; and/or
- further comprising, forming the provided training set; wherein the forming of the provided training set comprises generating a plurality of set of values for the physiological parameters, each set being generated in a vicinity of a reference value, and performing a simulation by the vascular model using the plurality of models corresponding to the generated plurality of set of values for the physiological parameters.

It is further provided a computer-implemented method of using the method discussed above. The method of use comprises obtaining the vascular model and the surrogate model, obtaining, from a user, the one or more measurements of the vascular behavior of the patient, calibrating the vascular model using the surrogate model and based on the one or more measurements, predicting the vascular behavior of the patient using the calibrated vascular model, and displaying, to a user, an avatar of a patient including a summary of the predicted vascular behavior. The method of use optionally further comprises providing, by a user, an updated value for one or more of the plurality of the physiological parameters, thereby further calibrating the vascular model.

It is further provided an inverse artificial neural network trained to predict physiological parameters of a patient given one or more measurements of a vascular behavior of the patient. The inverse artificial neural network may be trained according to the method discussed above.

It is further provided a computer program comprising instructions for performing the method and/or the method of use.

It is further provided a computer readable storage medium having recorded thereon the computer program and/or the trained inverse artificial neural network.

It is further provided a system comprising a processor coupled to a memory, the memory having recorded thereon the computer program.

It is further provided a device comprising a data storage medium having recorded thereon the computer program and/or the inverse neural network and/or the vascular model and/or the forward surrogate model.

The device may be portable. The device may form or serve as a non-transitory computer-readable medium, for example on a SaaS (Software as a service) or other server, or a cloud-based platform, or the like. The device may alternatively comprise a processor coupled to the data storage medium. The device may thus form a computer system in whole or in part (*e.g.* the device is a subsystem of the overall system). The system may further comprise a graphical user interface coupled to the processor.

## BRIEF DESCRIPTION OF THE DRAWINGS

Non-limiting examples will now be described in reference to the accompanying drawings, where:

- FIG. 1 shows a flowchart of an example of the method;
- FIG. 2 shows an example of a graphical user interface of a virtual arterial twin 3D viewer according to examples of the system;
- FIG. 3 shows an example of the system;
- FIG. 4 shows an example pipeline according to examples of the method;
- FIG. 5A shows an example forward physiology-inspired or a forward arterial neural network;
- FIG. 5B shows an example forward physiology-inspired neural network;
- FIG. 6A shows an example measurement reconstruction neural network;
- FIG. 6B shows an example inverse physiology-inspired neural network;
- FIG. 7 shows an example inverse physiology-inspired neural network including a measurement reconstruction neural network;
- FIG. 8A shows different types of sub-models;
- FIG. 8B shows an example of a 1D model;
- FIG. 8C shows an example of a 0D model; and
- Fig. 8D shows an example network of the plurality of vessels.

## DETAILED DESCRIPTION

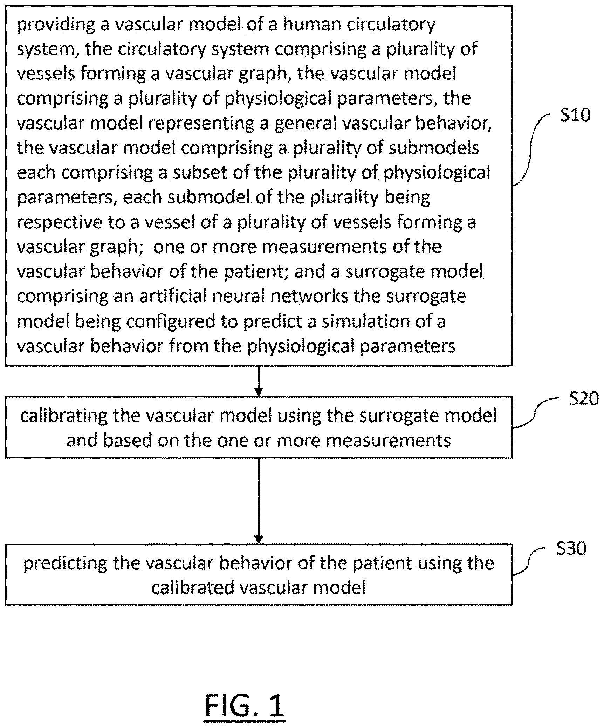

With reference to the flowchart of FIG. 1, it is proposed a computer-implemented method for predicting vascular behavior of a patient. The vascular behavior may be a hemodynamic behavior.

By "vascular behavior" it is meant a temporal and/or spatial description/representation of physiological dynamics in a system comprising a plurality of vessels. Said system may be, in examples, a human or animal vascular system. By a "patient" it is meant a human or animal subject to medical examination. A vascular model may comprise an avatar of the patient. As known *per se* an avatar may be a graphical representation (e.g., a 3D representation) of a subject, e.g., the body of the patient. An avatar may present a wireframe of said patient augmented with a 3D presentation of said plurality of vessels. By "vascular behavior of a patient" it is meant the vascular behavior of a (e.g., a human or an animal) body under medical examination. As known *per se,* "hemodynamics" refers to the movement/flow of blood. Such a movement may be described by a set of variables and according to general principles governing the flow of blood in the body. The set of variables may be a function of space and/or time with respect to a physical reference (e.g., with respect to an avatar when the vascular model includes an avatar) and a time reference of the vascular model, respectively. Thereby a respective value of each of the variables of the set of variables may have spatial and/or temporal dependency. The set of variables may (at least) comprise one or more variables related to the flow parameters (e.g., pressure or flow rate), one or more variables related to electrical signals of a body organ (e.g., heart's electrical activity), and/or one or more variables related to geometry of blood circulatory system (e.g., a section area of an artery). The hemodynamic behavior may then refer to a description of the hemodynamics by a set of values each for a variable of the set of variables. In examples, the set of variables may include variables that can be measured by a non-intrusive measurement device(s) and/ method(s), e.g., pressure cuffs, an MRI, ultra-sound measurements, a CT-Scan, and/or an ECG. Any of such devices or methods may provide a high fidelity, a medium fidelity, or a low fidelity measurements of a variable and at different levels of precision depending on a required application.

The method, in Step S10, comprises providing (S100) a vascular model of a circulatory system. By a "vascular model of a circulatory system" it is meant a model configured to describe said circulatory system, i.e., to predict a behavior of said system knowing some inputs. The circulatory system comprises a plurality of vessels forming a vascular graph. By a "circulatory system" it is meant an organ system (see https://en.wikipedia.org/wiki/Organ_system), i.e., a biological system, consisting of a group of organs amongst which a plurality of vessels that work together to circulate a fluid, or transfer a signal in a human or animal body. By a vessel it is meant a transfer channel for said fluid or signal. A vessel may be equivalently referred to as a vein or an artery hereinbelow. The circulatory system may form an open circuit (e.g., respiratory system) or a closed system (e.g., blood system). In examples where the vascular behavior is a hemodynamic behavior, the circulatory system may be a blood circulatory system that includes the heart and the blood vessels. The vascular graph of a circulatory system defines how the plurality of the vessels of the system are interconnected.

By "providing" an element it is meant obtaining said element by the method, e.g., from a user or (automatically or semi-automatically upon an action by said user) from a database. The vascular model comprises a plurality of physiological parameters and represents a general vascular behavior. By a "general vascular behavior" it is meant a pattern or trend of spatial and/or temporal variations in the vascular system. Thereby, the model may represent a general vascular behaviour by representing a spatial and/or temporal behaviour of one or more variables, for example by representing temporal oscillations of pressure and/or flow rate in one or more vessels of the plurality of vessels.

The vascular model comprises a plurality of submodels. Each submodel comprises a subset of the plurality of physiological parameters and is defined based on such a plurality of physiological parameters. Each submodel of the plurality of sub-models is respective to a vessel of a plurality of vessels. In other words, the plurality of submodels are interconnected according to the vascular graph which defines the relation between an output of a first submodel and an input of a second submodel. Each submodel may be configured to simulate a behavior of a respective vessel, thereby enabling the vascular model to simulate a behavior of the circulatory system.

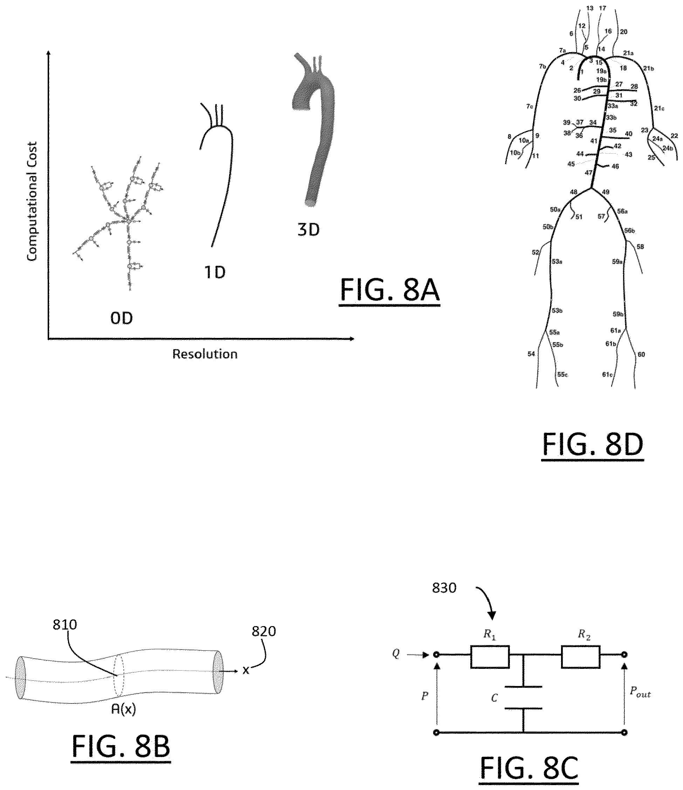

FIG. 8A presents different types of the sub-models. As discussed above, the vascular model comprises a plurality of submodels each respective to a vessel of a plurality of vessels. In examples, each sub-model may be a 0D, 1D, or 3D model of said respective vessel.

A 3D model may be based on a 3D geometry of the vessel and may provide a high-fidelity model based on 3D Navier-Stokes equations, fluid-structure interaction, and/or by considering coupled arterial and venous systems. A 3D model may solve for a vectorial partial differential equation (PDE) in time and 3D space in combination with a set of constitutive equations. In examples, such a 3D model may output, at each time instant, a 3D velocity field and/or a 3D pressure field.

A 1D model may be based on a 1D geometry of the vessel. In such models, variation of each variable in a vessel may only vary along said vessel, thereby the 1D model may not consider any variation over the section area. Such a 1D model may assume an axial symmetry in the vessel with fixed cylinder axis, i.e., dominance of axial velocity and thereby negligible orthogonal components, therefore merely considering radial displacements. A 1D model may provide a low fidelity model based on 1D Navier-Stokes equations, linearized behavior of materials (e.g., solid or fluid), and/or uncoupled systems with flow stationarity in the capillaries. Such 1D models may use linearized equations to simply and accelerate the method.

A 1D model may solve for a scalar PDE in time and 1D space in combination with a set of constitutive equations. In examples, such a 1D model may output, at each time instant, a flow rate, an area of the cross section of said vessel, and/or a pressure. An example of such a 1D model is presented in FIG. 8B, wherein the model is of the type:

$$
\begin{cases}
\dfrac{\partial A}{\partial t} + \dfrac{\partial Q}{\partial z} = 0 \\[2ex]
\dfrac{\partial Q}{\partial t} + \dfrac{\partial}{\partial z}\left(\alpha \dfrac{Q^2}{A}\right) + \dfrac{A}{\rho}\dfrac{\partial P}{\partial z} = -2\dfrac{\mu}{\rho}(\gamma_v + 2)\dfrac{Q}{A} \\[2ex]
P(A) = P_{ext} + \beta\left(\sqrt{\dfrac{A}{A_0}} - 1\right)
\end{cases}
$$

where $A$ is the section area 810, $Q$ is the flow rate, $t$ and $x$ are a time and a spatial coordination along the axis 820, and $P$ is the pressure. Here $P_{ext}$ is an external pressure, $A_0$ is a reference value of the section area and $\alpha$, $\beta$, $\gamma_v$, $\mu$, and $\rho$ are related to the physical parameters of the vessel and/or the fluid. In this model the set of variables may comprise the section area, the pressure, and the flow rate. Alternatively, the method may only consider two variables for the pressure and the flow rate and obtain the section area from the pressure using the last equation.

A 0D model may be based on a lumped parameter modeling which simplifies the description of the (spatially distributed) circulatory system into a topology consisting of discrete entities that approximate the behaviour of said system under certain assumptions (see https://en.wikipedia.org/wiki/Windkessel_effect). A 0D model may solve for scalar ordinary differential equation (ODE) in time in combination with a set of constitutive equations. In examples, such a 0D model may output, at each time instant, a flow rate for the vessel, a volume for the vessel, and pressure for the vessel. The method may model one or more vessels using a 0D model as presented in FIG. 8C. The method may in particular use the 0D models as a boundary condition to represent a group of small vessels connected to the vessels under study. In such a model, a vessel or a group of vessels may be modeled by an electric circuit 830, for example of a type

$$\left(1 + \frac{R_1}{R_2}\right) Q(t) + C R_1 \frac{\partial Q}{\partial t} = \frac{P(t) - P_{out}}{R_2} + C \frac{\partial P}{\partial t}$$

where $C$, $R1$, and $R_2$ are related to the physical parameters of the vessel and/or the fluid, $Q(t)$ and $P(t)$ are the flow rate and pressure values, and $P_{out}$ is the output pressure value.

The method further in Step S10 comprises providing (S102) one or more measurements of the vascular behavior of the patient. The method may obtain the one more measurement according to any non-intrusive known methods as discussed above optionally via an intervention by a user.

The method further comprises in Step S10 providing (S104) a surrogate model also known as a forward model. As known (see https://en.wikipedia.org/wiki/Surrogate model) a surrogate model is a substitute model configured to approximate an output of a more complex model or simulation. The surrogate model is configured to predict a simulation of a vascular behavior from the physiological parameters. Thereby, the provided surrogate model may be the substitute model for the (more complex) plurality of submodels and to approximate a general vascular behavior represented by the vascular model. By "predicting a simulation" it is meant obtaining as estimation of said simulation. The surrogate model comprises a plurality of artificial neural networks. Each artificial neural network represents a vessel of the plurality of vessels and may correspond to a submodel of the plurality of submodels. Hereinafter, an artificial neural network may be equivalently referred to as a neural network. The artificial neural networks are connected to each other following the vascular graph. By "following the vascular graph" it is meant that a connectivity among the plurality of artificial neural networks and thereby input/output of each artificial network is according to the vascular graph of the plurality of vessels.

The method further comprises, in Step S20, calibrating the vascular model using the surrogate model and based on the one or more measurements. By "calibrating the vascular model" it is meant obtaining a value (for each) of the plurality of physiological parameters. Each value obtained upon the calibration is associated to one of the one or more physiological parameters. Such an obtention may be or comprise an update of one or more existing values of the plurality of physiological parameters. In examples, the method may create any vascular with default value for the plurality of physiological parameters and then updates them using the surrogate model and based on the one or more measurements to be adapted to the patient. Alternatively or additionally, the method may assign a vascular model to a patient and update the value of one or more of the physiological parameters as the physiology of said patient changes, for example due to aging.

FIG. 8D presents a plurality of vessels according to 1D ADAN56 network which is an example of Anatomical Detailed Arterial Networks (ADAN). Such ADAN has been introduced, for example, in articles Blanco et al., Blood flow distribution in an anatomically detailed arterial network model: criteria and algorithms. Biomech. Model. Mechanobiol. 13 (2014) 1303-1330, and Blanco et al., An anatomically detailed arterial network model for one dimensional computational hemodynamics. IEEE Trans. Biomed. Eng. 62 (2015) 736-53, which are incorporated herein by reference. ADAN56 showed in FIG. 8D is a network consisting of 77 arteries.

The method further comprises, in Step S30, predicting the vascular behavior of the patient using the calibrated vascular model. By a "prediction of the vascular behavior" it is meant an approximation, e.g., a numerical approximation, of said behavior over a time period. In other words, the surrogate model is configured to output an approximation of the (general) vascular behavior represented by the vascular model. Such an approximation defines a variation profile of said behavior over said period.

The time period may be set by a user. Alternatively or additionally the time period may be automatically set long enough to record a number of periodic behaviour in the circulatory system, for example at least one complete period of the blood flow (i.e., one cardiac cycle).

The method thereby constitutes an improved solution as the vascular model is adapted to the particular behavior of a patient by setting its parameter according to the given one or more measurements. Such a patient-specific adaptation improves the precision of the method in predicting the behavior. Furthermore, such an adaptation is available only from a given subset one or more measurements and not necessarily the provided measurement of the vascular behavior in the plurality of vessels. This constitutes an improved solution by enabling the calibration by less required measurements. In examples, the method may only need to have the value one or more measurements which can be obtained using non-intrusive measuring methods. This facilitates the application of the method by removing the need to expensive, time-consuming, and/or intrusive measurements.

Furthermore, the method is configured to predict the behavior represented by the vascular model. In other words the method is able to approximate the behavior of the circulatory system over the whole period of a duration of interest. Such an approximation provides significantly more information about the pathological condition of a patient compared to outputting quantities of interest which are merely a functional of said behavior (e.g., an average over said period). This improves the medical decisions based on the predictions provided by the method.

In examples, the surrogate model may comprise a plurality (e.g., more than one) of artificial neural networks. In such examples the artificial neural network discussed above may consist of the plurality of artificial neural networks. Each artificial neural network of the plurality may then be respective to a vessel of the plurality of vessels. The artificial neural networks of the plurality may be connected to each other following the vascular graph. In such examples, an artificial neural network (of the plurality of artificial neural networks) may be in correspondence with a submodel (of the plurality of submodels) while both are respective a same vessel (of the plurality of vessels). The surrogate model of such examples may be called a physiology-informed or equivalently an arterial artificial neural network as its structure follows the vascular graph.

In examples, the predicting of the vascular behavior using the calibrated vascular model may comprise performing a simulation by the calibrated vascular model using the plurality of submodels. In other words, in such examples each submodel may obtain a simulation of the respective vessel so as to form a simulation of the vascular behavior using the plurality of the sub-models in the vascular model arranged as the vascular graph. In other examples, the predicting of the vascular behavior using the calibrated vascular model may comprise predicting a simulation by the calibrated vascular model using the surrogate model. The method may obtain such a prediction by applying the surrogate model on the physiological parameters obtained or updated in the calibrating step.

In examples, the one or more measurements may comprise one or more pressure values and/or one or more flow rate values and/or one or more cross-sectional area values of the plurality of vessels. In particularly efficient examples, the one or more measurement may consist of one or more pressure values and one or more flow rate values. In such examples, the method may obtain the respective cross sectional area values using a respective value of the pressure.

In examples, said physiological parameters comprise at least one or more of mechanical properties of the plurality of vessels, and/or geometrical properties of the plurality of vessels. The mechanical properties of the plurality of vessels may comprise a respective Young's modulus for the vessels. In examples where the vascular behavior is a hemodynamic behavior the mechanical properties may further comprise a density and/or a viscosity of a blood. The geometrical properties may comprise a length, an inner/outer radius, and/or wall thickness of the vessels. Alternatively or additionally, the physiological parameters may comprise a function or combination of the geometrical and mechanical parameters. Such a function may be an explicit or implicit function of multiple mechanical and/or geometrical parameters. An example of such a function is as the following:

$$R_1 = \sqrt{\frac{\rho E h}{2\pi^2 r^5 (1 - v^2)}}$$

which represents a resistor parameter $R_1$ in a 0D model as discussed above. While the parameter $R_1$ depends on other geometrical parameters (e.g., radius $r$) or mechanical parameters (e.g., the Young's modulus $E$) the method may use parameter $R_1$ as an independent parameter to reduce the number of physiological parameters.

In examples, the calibrating of the vascular model may comprise providing an inverse artificial neural network trained to predict the physiological parameters of the patient given one or more measurements of the vascular behavior of the patient. By the "inverse artificial neural network" it is meant an artificial neural network configured to solve (at least partially) an inverse problem. As known *per se* in the field of applied mathematics and engineering, an inverse problem is a process of calculating from a set of observations the causal factors that produced them. In particular, an inverse problem in the field of predicting vascular behavior of a patient may comprise determining a value of the plurality of physiological parameters of a vascular model given one or more measurements of the vascular behavior of the patient. The given one or more measurement may be a subset of the provided one or more measurements in Sub-step S102 of Step S10. By "an inverse artificial neural network trained to predict the physiological parameters of the patient given one or more measurements of the vascular behavior of the patient" it is meant an artificial neural network configured to output the physiological parameters of the patient when inputted by one or more measurements of the vascular behavior of the patient. Such a neural network is trained (i.e., learnt) on a training dataset that comprises a plurality of physiological parameters of patients in correspondence to a respective plurality of the one or more measurement of the vascular behavior of the patient. More details of such a dataset are discussed below.

The calibrating of the vascular model may then comprise determining a value of the plurality of physiological parameters using the provided inverse artificial neural network. Being provided with the inverse artificial network discussed above, the determining a value of the plurality of physiological parameters may include applying the provided inverse artificial network on the provided one or more measurements of the vascular behavior of the patient. Such an application may output a value for the plurality of physiological parameters specified to a patient corresponding to the one or more measurements of the vascular behavior of said patient, thereby calibrating the vascular model of said patient.

In examples, the provided inverse artificial neural network may consist of a plurality (i.e., more than one) artificial neural networks. Each artificial neural network of said plurality may represent a vessel of the plurality of vessels. The artificial neural networks may be connected to each other following the vascular graph. In such examples, the method may determine a value of the plurality of physiological parameters using the provided plurality of inverse artificial neural networks.

In examples, the method may comprise, prior to the providing of the inverse artificial neural network, training the inverse artificial neural network. The training of the inverse artificial neural network may comprise providing a dataset of patients comprising a plurality of entries. Each entry in said database may comprises the plurality of physiological parameters being in correspondence to a simulation result of a patient vascular behavior. Said simulation result may be computed with the physiological parameters. The method may then train the inverse artificial neural network based on the dataset. The training may be according to any known methods in the field of machine-learning. The training may comprise minimizing a loss function. Examples of such a loss function are discussed below.

The provided dataset may additionally comprise respective physical features for each entry. By physiological features it is meant the features which characterize an individual patient, e.g., age, weight, height, sex. The physiological parameters may reflect the physiological features. As known *per se*, such physiological features have an impact on a patient's physiological parameters such as mechanical behavior of arteries, and/or geometrical properties, for example a tall person has longer arteries than a shorter person.

In examples, the inverse artificial neural network comprises a first part and a second part. The first part may be configured to accept as input a subset of the one or more measurements of the vascular behavior of the patient. Such a subset of the one or more measurements may be equivalently referred to partial measurements. Said first part may be configured to output (i.e., to compute) a reconstruction of the one or more measurements of the vascular behavior of the patient. In other words, a first part of the inverse artificial neural network may be configured to reconstruct the whole of the one or more measurements required to calibrate the vascular model from the partial measurements. The second part of the inverse artificial neural network may be configured to accept as input such a reconstruction of the one more measurements and to output the physiological parameters.

In examples, the second part may comprise one artificial neural network, or a plurality of artificial neural networks being connected to each other following the vascular graph. In examples where the second part may comprise one artificial neural network the method is able to optimize the training of such an inverse neural network as the number of weights needed to be found is reduced. On the other hand, the examples in which the second part comprises a plurality of neural networks connected to each other following the vascular graph enables the method to refine the calibration by training an inverse network which has a same general connectivity in its architecture as the surrogate model by following the vascular graph.

The training of the inverse artificial neural network based on the dataset may comprise minimizing a first discrepancy between the one or more measurements of the vascular behavior of the patient; and a prediction of a vascular behavior simulation using the surrogate model and based on the predicted physiological parameters by the provided inverse artificial neural network. Minimizing such a first discrepancy may train the inverse neural network, i.e., determine weights of the inverse neural network, so as to minimize a prediction error of the forward surrogate model based on the determined value of the plurality of physiological parameters. In other words, minimizing the first discrepancy tends to decrease the prediction error of the forward surrogate model by determining the respective optimized plurality of physiological parameters. Specifically, the first discrepancy may be of the form:

$$\left\| d - f(NN^{inv}(d)) \right\|$$

where d denotes the one or more measurements (upon a reconstruction of the partial measurements), $NN^{inv}(d)$ denotes the application of the inverse neural network $NN^{inv}$ on the one or more measurement, $f$ is the forward surrogate model, and $f(NN^{inv}(d))$ denotes the prediction of a vascular behavior simulation using the forward surrogate model. Here $\|.\|$ denotes a distance, for example a time integral of a sum of absolute value of difference between a measurement of the one or more measurement $d_i$, and a respective prediction $f_i(NN^{inv}(d))$.

Alternatively or additionally, the training of the inverse artificial neural network based on the dataset comprises minimizing a second discrepancy between the physiological parameters reflecting the physiological features, and the predicted physiological parameters by the provided inverse artificial neural network. Minimizing such a second discrepancy may train the inverse neural network, i.e., determine weights of the inverse neural network, so as to minimize an error of the inverse artificial neural network in determining a value of the plurality of physiological parameters. In other words, minimizing the second discrepancy tends to decrease the determination error of the inverse artificial neural network by determining the respective optimized plurality of physiological parameters. Specifically, the second discrepancy may be of the form:

$$\left\| NN^{inv}(d) - p \right\|$$

where $p$ designates the plurality of physiological parameters, $d$ is the one or more measurement, $NN^{inv}$ is the inverse artificial neural network, and $NN^{inv}(d)$ is the physiological parameters determined from the one or more measurements using the inverse artificial neural network.

In examples, the training of the artificial neural network based on the dataset comprises minimizing a first weighting of the first discrepancy and a second weighting of the second discrepancy. The training may for example minimize a sum said first weighting of the first discrepancy and said second weighting of the second discrepancy. Specifically, the training of the artificial neural network based on the dataset comprises minimizing a loss function $L$ of the form:

$$L = \left\| NN^{inv}(d) - p \right\| + \alpha \left\| d - f\left( NN^{inv}(d) \right) \right\|$$

where each of the first discrepancy and the second discrepancy is according to the specific form discussed above, the first weighting is equal to unity and the second weighting is equal to $\alpha$ which may be equivalently called regularization parameter. The regularization parameter may be of strictly positive value and be set to ensure a balance between the two terms of the loss function, e.g., being of a same order of magnitude. This improves the minimization of the loss function, i.e., in terms of the iterations needed to be performed in an iterative minimization and/or in terms of the computation time. For example, the regularization parameter may be a value between 1 and 10, or preferably 5, or 10.

In examples, the method may further comprise, prior to the providing of a surrogate model, training the surrogate model. The model may thereby comprise providing a training dataset, each entry in the training dataset comprises a value for the physiological parameters, and a value for a simulation by the vascular model using the plurality of models. The method may then train the surrogate model using the training dataset. The providing of the training dataset may comprise obtaining a dataset stored on a local database or a remote database (e.g., on a cloud). The training database may be a database comprising clinical studies of a number of patients, for example, 1000, 10000 or 100000 patients. In particular, the method may obtain a training dataset designed for such a purpose comprising a statistically satisfactory variety of physiological features of the patients, i.e., a variety of sex, age, height, weight, body mass, or a variety of underlying diseases like diabetes, and/or high blood pressure. The training dataset may alternatively be a tailor-made dataset for a particular problem at hand, for example a training dataset may only include clinical data in an age range of children, teenagers, or old people.

Alternatively, at least a part of the training dataset may be a synthetic dataset. The synthetic dataset may be formed by a dataset forming method. In examples, the method for predicting vascular behavior may comprise a dataset-forming method.

In examples, the dataset-forming method may comprise generating a plurality of set of values for the physiological parameters. Each of said set may be generated in a vicinity of a reference value of physiological parameters. Such a reference value may be a value of a benchmark for which detailed experimental or computational benchmarks of vascular behavior are available. The vicinity of a reference value may be an interval of variation for the value of each of the plurality of physiological parameter around the refence value. The interval of variation may allow a variation below 20%, or below 10%. The dataset-forming method may vary each parameter in the interval randomly or according to any known methods in the field, e.g., a Latin hypercube. The dataset-forming method may then comprise performing a simulation by the vascular model using the plurality of models corresponding to the generated plurality of set of values for the physiological parameters.

Upon providing of the dataset or as a step in forming the dataset, the method may apply further steps of post-processing of the dataset before using the dataset. Such steps may comprise a normalization step on the data, and/or non-dimensionalization of the data. The non-dimensionalization and/or normalization of the data, for example pressure or flow rate data, may comprise dividing the data by a respective maximum value (e.g., a maximum systolic value) across the dataset for each artery. In examples, the method may normalize the input parameters to be in the unitary hypercube. This helps retain the physics of the problem, and the scales of the input/outputs. Such post-processing steps improves the training of the neural network and the precision of the prediction by such trained networks.

The method discussed above generally manipulates a vascular model of a patient. Such a vascular model may form or be a part of a digital twin of said patient. A digital twin of a patient is defined as a virtual representation (i.e., "digital twin") of a patient (i.e., "physical twin"). Such a virtual representation may further comprise a graphical representation, in combination with a plurality of parameters (e.g., physical, physiological, pathological, etc.) related to the patient. Additionally, the digital twin may comprise a surrogate model as discussed above. Such a surrogate model may constitute a less complex substitute for a more complex but more precise vascular model. As known in the art, see for example, Venkatesh et al., Health digital twins as tools for precision medicine: Considerations for computation, implementation, and regulation. *npj Digit. Med.* 5, 150 (2022), with appropriate use, such digital twins are able to model random perturbations on the digital twin to gain insight into the expected behavior of the physical twin offering applications in precision medicine, clinical trials, and public health. It is also provided a computer-implemented method of using the method for predicting vascular behavior of a patient as discussed above. Such a method may be used for providing a vascular twin of said patient. Such a method may be equivalently referred to hereinbelow as the "twin patient method". As discussed above, providing a vascular twin is beneficial in providing a tool, e.g., for diagnosis of the patient, to the medical practitioners. The twin patient method comprises obtaining the vascular model and the surrogate model as discussed above. The method then obtains, by a user (e.g., a medical practitioner/medical doctor/nurse), the one or more measurements of the vascular behavior of the patient. In examples, said user may input the one or more measurements of the vascular behavior of the patient directly. Alternatively or additionally, the method may import the one or more measurements of the vascular behavior of the patient from a patient database upon an input of a patient identifier (ID) by said user. In examples, the patient ID may comprise one or more of a last name, a first name, a registration ID at a clinic/hospital, an insurance number and/or a date of birth. Said patient database may be a local database stored on a local hard drive or a database stored on a cloud.

The twin patient method then calibrates the vascular model using the surrogate model and based on the one or more measurements. The twin patient method performs such a calibration as discussed above. The method then predicts the vascular behavior of the patient using the calibrated vascular model, and displays, to a user, an avatar of a patient including a summary of the predicted vascular behavior. The summary may comprise profile of the physiological parameters in the plurality of vessels, like a pressure profile over time or a flow rate profile over time. The summary may also comprise the physiological features of a patient, like name, or age.

In examples, the vascular twin method may further comprise providing, by a user, an updated value for one or more parameters of the plurality of the physiological parameters. Upon such a providing of an updated value for one or more of the plurality of the physiological parameters the method re-performs the calibration of the vascular model as discussed above.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g., one for the program, and possibly one for the database).

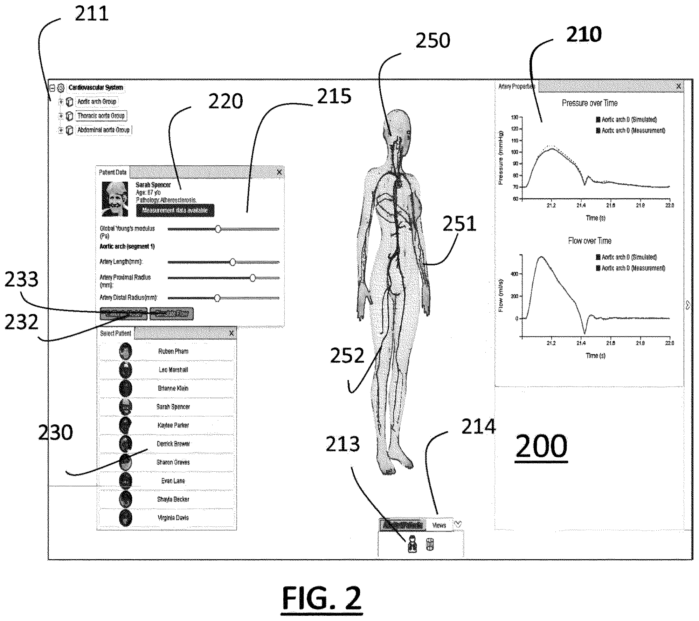

FIG. 2 shows an example of the GUI 200 of the system, wherein 250 may be an avatar of a patient. The GUI 200 shows of a prototype application of an arterial virtual twin experience. The GUI 200 may be a typical interface, having standard menu bars 211, as well as bottom and side toolbars 214, 215. Such menu- and toolbars contain a set of user-selectable icons, each icon being associated with one or more operations or functions, as known in the art. Some of these icons are associated with software tools, adapted for editing and/or working on the 3D avatar 250 displayed in the GUI 200.

The GUI 200 may further show various types of graphic tools like the ones grouped in 213 for example for facilitating 3D orientation of the 3D avatar 250. Any action via the GUI may be controlled by a haptic device to allow the user to interact with the graphic tools. In particular, the user may be able to move, rotate, or scale the 3D avatar 250 using a haptic input device like a touch pad, or a mouse optionally in combination with a keyboard, as known in the art.

In example, an application of the virtual twin method may start by performing the Step S10 discussed above by selecting a patient in a menu 230 in GUI 200, for example by a mouse selection or using a search functionality by first name, last name, and/or or other identifications. The method, optionally via a further intervention of the user, may assign to each patient, depending on their physical and physiological characteristic (e.g., age, sex, weight, height, etc.) an avatar 250 to be personalized with their available measurement data. The avatar 250 includes a wireframe 251 representing a schematic volume of said patient and a blood circulatory system 252. The avatar may display separately (e.g., in a different color) the arteries in which the one or more measurements of the vascular behavior is provided and the rest of arteries for which such measurements have been reconstructed via the calibrating of the vascular model. The blood flow measurements in the selected arteries can be analyzed prior to the personalization. The summary displayed to the user includes a pressure and flow rate profile 210 and information about the patient 220 (e.g., name, age, pathology). The user may select one of the vessels presented in the blood circulatory system 252 (e.g., upon a mouse click). Upon such a selection, the provided one or more measurements of the vascular behavior of the patient may be displayed in the summary 210. Furthermore, upon such a selection, the method may display a value of one or more physiological parameters of said vessel in the summary 220, for example, a Young's modulus, a length of the vessel or a distal radius of the vessel. The user may be able to update the displayed values, e.g., either graphically (e.g., by moving a respective bar on the GUI 200) or by entering a new respective value. The user may then (re-)calibrate the model based on said updated values of the one or more measurements for example by clicking the calibration button 232 in GUI 200. Upon, such a (re-)calibration, the method may automatically predict the vascular behavior of the patient (i.e., predicting a simulation as discussed above) using the (re-)calibrated vascular model and display an updated result in the summary 210. Additionally, the method may perform a simulation by the calibrated vascular model using the plurality of models upon the user selection, for example by clicking the simulation button 233 in GUI. This availability of performing simulations constitutes an improved solution which enables obtaining more precise predictions of a vascular behavior when a more accurate prediction of said behavior needs to be provided.

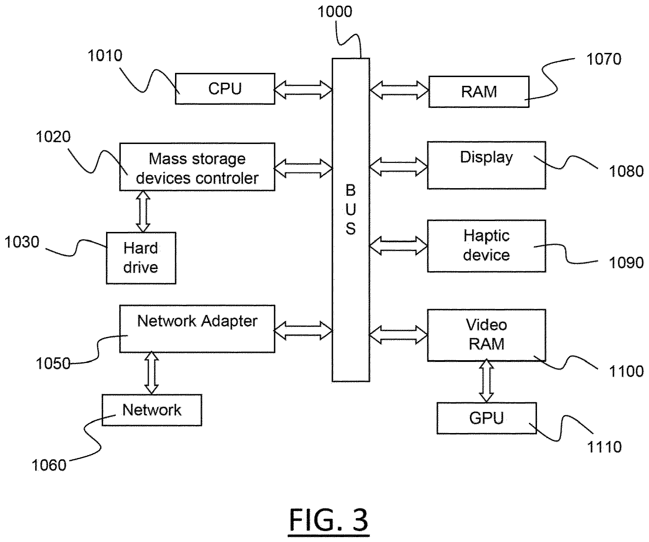

FIG. 3 shows an example of the system, wherein the system is a client computer system, e.g. a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

Implementations of the examples of the methods discussed above are now presented.

The implementations are related to the field of physics simulation, and more specifically to simulating fluid dynamics. Particular implementations are discussed below related to hemodynamics use-cases. The implementations, however, may be related and may be applied in other vascular scenarios where measuring the model parameters of the entire system is not practicable.

Simulating the entire 3D hemodynamics system is time consuming in terms of computational power. Even in the cases the simulation is possible, obtaining the boundary conditions in a non-intrusive way is a hard task, since clinical data are usually incomplete. The implementations are more specifically related to a neural network-based inverse problem solving approach to identify a patient-specific set of 1D-0D model parameters from measurement data.

The implementations establish a novel approach for simulating a patient's arterial blood pressure and flow rate over a cardiac cycle via surrogate modeling of a 1D-0D arterial system. The implementations constitute a solution to personalize this model, i.e., providing a patient-specific model, by calibrating against clinical measurements of the patient's anatomical and physiological features.

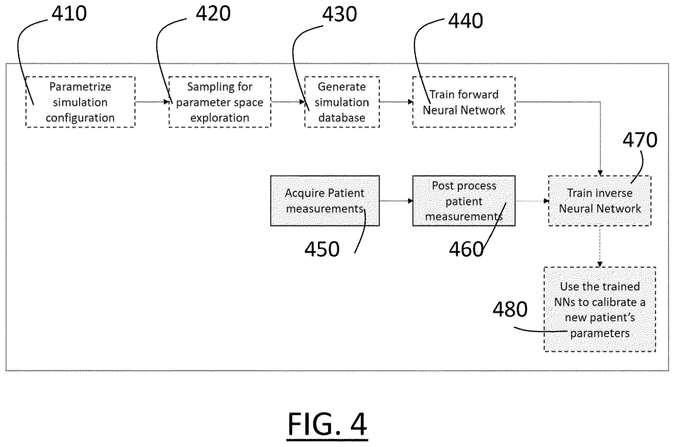

The implementations follow an approach according the following pipeline which is depicted in FIG. 4 in line of the steps S10-S30 discussed above:

- In step 410, a parametrized model of the 1D-0D arterial system is constructed with physiological/geometrical parameters.
- In step 420, physiological variation intervals are specified for each model parameter. The parameters are sampled in the parameter design space in order to construct a simulation database of N patients.
- In step 430, the database is generated by solving N simulations according to the sampled sets of model parameters with a hemodynamics solver.
- In step 440, a "forward" neural-network surrogate model of the arterial blood flow simulation is constructed and trained on the generated database. The neural network is able to determine the blood flow behavior in the arterial system given the patients anatomical and physiological features. In variations, architecture of the neural network may be inspired by the physiology of the arterial system according to arterial neural network discussed above.
- In steps 450 and 460, patient blood flow data is acquired in a few arteries, and the data is pre-processed to resemble the simulation data.
- Given any new patient, the goal is to determine the best set of model parameters that would generate a simulation that close to their measurement data. For that, an inverse problem-solving method is applied. In step 470, the inverse problem is solved by training a second neural network, that, for a given set of measurement data, is able to determine the best set of model parameters. It is trained by using the previously trained "forward" surrogate model.

In step 480, and once a patient's best set of model parameters is determined, their hemodynamics can be computed either by using the forward neural network, or by solving the 1D-0D simulation with the hemodynamics solver.

The surrogate model of the implementations takes a fraction of the time taken by a full simulation. This speedup enables using hemodynamics simulation in more complex workflows, such as patient specific calibration. Furthermore, the neural network (of the inverse problem) can be trained in a way that only a subset of the patient data is required to estimate the entire hemodynamics system. The surrogate model has the same outputs as the simulation and not just a macroscopic quantity of interest (e.g., a function of the outputs).

The implementations constitute a novel approach for simulating a patient's arterial blood pressure and flow rate over a cardiac cycle via surrogate modeling. The physiology-inspired neural network surrogate model gives, for a set of arterial model parameters, functional outputs of the pressure and flow rate signals for each artery of the system. This surrogate model also provides a novel neural network-based inverse problem solving approach. Its aim is to identify a patient-specific set of 1D-0D model parameters from a limited set of measurement data. As discussed above, applications of the implementations may be much wider than hemodynamics simulation, because the developed techniques, i.e., neural-network based surrogates and inverse-problem solving method, can be applied to a very diverse set of fields and applications.

The construction of the forward surrogate model in the implementations is now discussed. The implementations construct the closest possible prediction of the simulation's actual outputs. In other words, instead of outputting macroscopic output values (like average values), the implementations are configured to output pressure, flow rate and cross sectional area over a complete cardiac cycle at the central location of each artery. This requires a surrogate model with functional outputs. Since the cross-sectional area is a function of the blood pressure, we can limit the problem to the first two signals.

In order to ameliorate the predictions related to the arterial flow of a given patient by the surrogate model, the implementations employ training simulation dataset accounting for all of the physiological factors (i.e., features) that may vary physiological parameters of the arterial system, such as age, gender, height, and/or weight. Physiological variation intervals are therefore specified for each model parameters, and a pressure/flow rate signal dataset can then be constructed via Latin-hypercube sampling and hemodynamics simulation.

The implementations construct an artificial neural network which acts as the forward surrogate model. The artificial neural network is composed of an input layer of all the physiological parameters that characterize a patient. The artificial neural network further comprises a plurality of hidden layers, for example five (e.g., fully connected) hidden layers of four neurons per layer. The implementations use tanh (i.e., hyperbolic tangent) as the activation function. The artificial neural network further comprises a linear output layer that represents the hemodynamics behavior of each artery according to the hemodynamics simulation database. Each artery (of the database) is represented by vectors of each hemodynamics field (e.g., pressure, flow rate) over a full cardiac cycle with a value for each time step of the cardiac cycle. The implementations then train the artificial neural network by Adam optimizer.

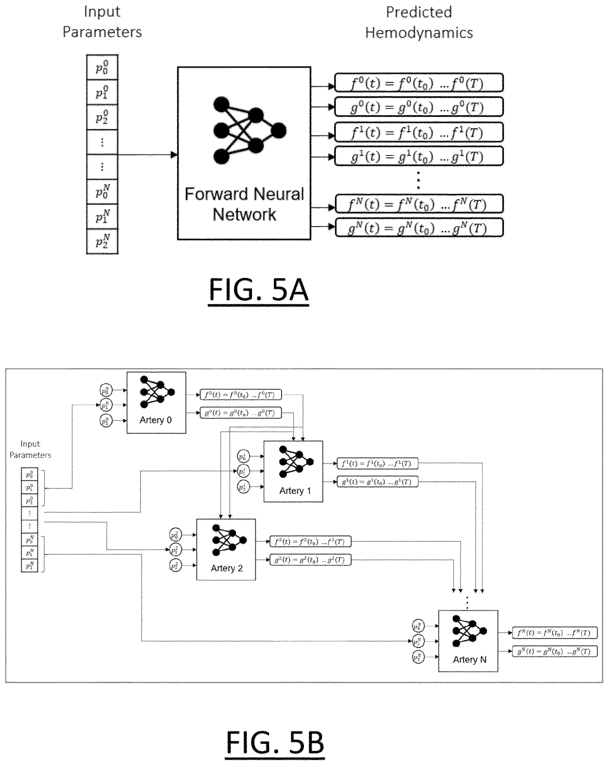

FIG. 5A presents an example of a neural network according to such implementations. In FIG. 5A,

$$p_0^0, p_1^0, p_2^0, \ldots, p_0^N, p_1^N, p_2^N$$ are the input parameters (e.g., the physiological parameters related to N arteries) and $f^0(t), g^0(t), f^0(t), g^0(t), \ldots, f^N(t), g^N(t)$ are the predicted hemodynamics in the N arteries (e.g., a pressure and a flow rate) in a time interval between $t_0$ and $T$.

In some arterial variations as discussed above, the implementations employ a decomposition of the problem into N individual neural networks in charge of predicting the flow signals of each artery given its parameters. Here N signifies the number of arteries in the system.

FIG. 5B presents an example of a neural network according to such implementations. The implementations may enumerate the plurality of individual neural networks following the physiology of the arterial system, such that an artery of an upstream has a smaller index than any artery of a relative downstream. The prediction of artery n is then fed as an additional input to the neural network of artery n+δ when δ in an integer larger than 1, following the physiology of the arterial system. The direction of the arrows in FIG. 5B schematically represents the flow direction and thereby the direction of passing the information through the graph. The implementations can adapt the architecture and hyperparameters of each artery neural network so as to predict the behavior of each specific artery of a particular complexity. The predicted behavior information is passed through the graph in order to provide an additive conformal system prediction.

The construction of the neural network which solves the inverse problem is now discussed. The implementations determine the best set of model parameters to fit a patient's blood pressure (and thus cross-sectional area) and flow rate signals over a full cardiac cycle based on the "inversion" of the forward physiology-inspired neural network.

The previously trained "forward" surrogate model is able to emulate the simulation solver that usually takes several minutes to compute. For an input of model parameters, the prediction of the pre-trained model consists of N (#arteries) x T/dt (#time steps) x number of Quantities of Interests (QoIs) (i.e., pressure P and flow rate Qsignals).

The implementations then, define the inverse problem as to obtain a neural network, that takes M blood flow signals, and outputs the 1D-0D model parameters that are necessary to reconstruct the patient's N artery blood flow signals (with a "fit" of the available M arteries, and a "guess" of the missing N-M arteries). The same simulation data set based on synthetic patients is used to train this new model.

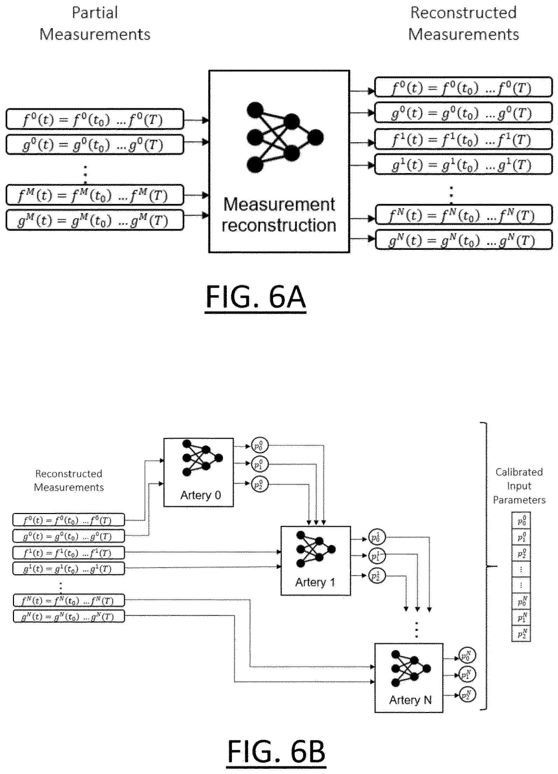

An example of a measurement reconstruction neural network is depicted in FIG. 6A. The measurement reconstruction neural network 610 is configured to learn the correlations between the known and the missing signals. In other words, the measurement reconstruction neural network 610 receives M signals forming partial measurements 620 (an M<N) to reconstruct (i.e., predict) remaining N-M missing signals to obtain the reconstructed measurements 630 comprising N signals. These N signals are used to predict the parameters with an additional neural network structure.

FIG. 6B presents an example of such an additional neural network structure 650 which receives the reconstructed measurements 630 and to output the calibrated output parameters 640. Similar to the structure 500 in FIG. 5B, the proposed neural network structure 650 relies on the decomposition of the problem into N individual neural networks, in charge of predicting the artery parameters of each artery individually. The prediction of the parameters of artery n is then fed as an additional input to the neural network of artery n+δ when δ in an integer larger than 1, following the physiology of the arterial system. The architecture and hyperparameters of each "artery neural network" can be adapted, so that it can predict the complexity of each specific artery.

The predicted information is passed through the graph in order to provide an additive conformal system prediction. The training loss function $L$ is constructed as:

$$L = \left\| NN^{inv}(d) - p \right\| + \alpha \left\| d - f(NN^{inv}(d)) \right\|$$

where f is the pre-trained forward neural network, $NN^{inv}$ is the inverse neural network, d the hemodynamics data, et p the parameters that are predicted, and $\alpha$ a relaxation parameter.

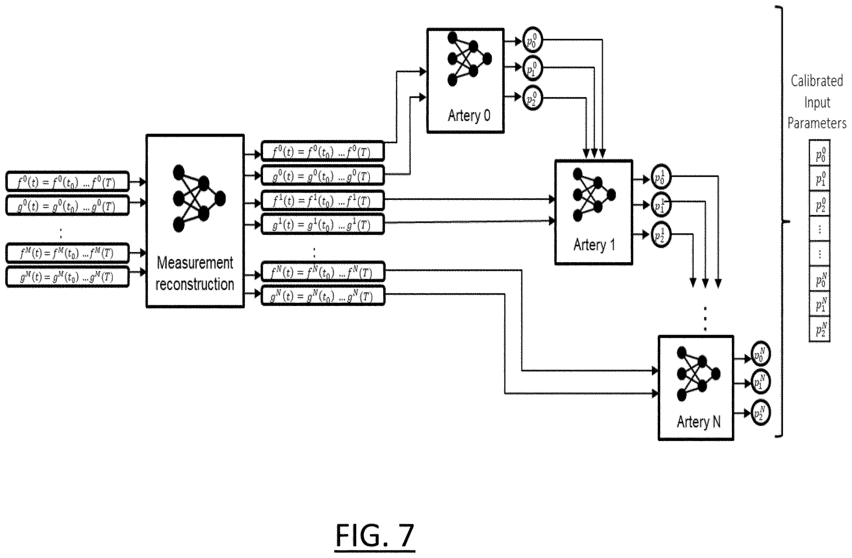

FIG. 7 shows an example of how the measurement reconstruction neural network is connected to the additional neural network.

As an example of how the calibration process may be integrated into an application, a prototype of an arterial virtual twin experience according to the implementations is now discusses. The prototype starts by assigning to each patient, depending on their demographics, an avatar which is personalized with their available measurement data. The prototype may analyze the blood flow measurements in the selected arteries prior to the personalization.

Simulating the arterial blood flow of a single patient usually takes several minutes. A real time experience can be achieved by using our pre-trained forward surrogate model. And thanks to that, the virtual twin can instantaneously predict the behavior of the whole arterial system, and for any given patient. To make sure that the model parameters are personalized to fit the patient's unique features, the inverse neural network is used to determine the best set of anatomical and physiological parameters given the patient's measurement data. Each blood vessel is therefore a reflection of the patient's real body features. The patient's blood pressure and flow rate predicted with the real time experience can be compared to the clinical data.

The virtual twin can augment reality by giving us access to the blood flow even on unmeasurable arteries, therefore enhancing the practitioner's capabilities for diagnosis and interventional planning. The arterial twin can also provide patient-specific boundary conditions for future full 3D simulations of any given part of the cardiovascular system, for example the carotid artery.

**Claims**

1. A computer-implemented method for predicting vascular behavior of a patient, in particular a hemodynamic behavior, the method comprising:

    - providing (S10):

        -- a vascular model of a circulatory system, the circulatory system comprising a plurality of vessels forming a vascular graph, the vascular model comprising a plurality of physiological parameters, the vascular model representing a general vascular behavior, the vascular model comprising a plurality of submodels each comprising a subset of the plurality of physiological parameters, each submodel of the plurality being respective to a vessel of a plurality of vessels;
        -- one or more measurements of the vascular behavior of the patient; and
        -- a surrogate model comprising an artificial neural network, the surrogate model being configured to predict a simulation of a vascular behavior from the physiological parameters;

    - calibrating (S20) the vascular model using the surrogate model and based on the one or more measurements; and
    - predicting (S30) the vascular behavior of the patient using the calibrated vascular model.

2. The method of claim 1, wherein the surrogate model comprises a plurality (N) of artificial neural networks, each artificial neural network of the plurality being respective to a vessel of a plurality of vessels, the artificial neural networks of the plurality being connected to each other following the vascular graph.

3. The method of any of claims 1 to 2, wherein the calibrating of the vascular model comprises:

    - providing an inverse artificial neural network trained to predict the physiological parameters of the patient given one or more measurements of the vascular behavior of the patient; and
    - determining a value of the plurality of physiological parameters using the provided inverse artificial neural network.

4. The method of claim 3, wherein the method, prior to the providing of the inverse artificial neural network, comprises training the inverse artificial neural network; the training comprising:

    - providing a dataset of patients, each entry in said database comprising the plurality of physiological parameters being in correspondence to a simulation result of a patient vascular behavior, the simulation result being computed with the physiological parameters, the physiological parameters reflecting the physiological features; and
    - training the inverse artificial neural network based on the dataset.

5. The method of any of claims 3 to 4, wherein the inverse artificial neural network comprises:

    - a first part configured to accept as input a subset of the one or more measurements of the vascular behavior of the patient and to output a reconstruction of the one or more measurements of the vascular behavior of the patient; and
    - a second part configured to accept as input the reconstruction of the one or more measurements of the vascular behavior of the patient, and to output the physiological parameters; wherein the second part optionally comprises:
    - one artificial neural network, or
    - a plurality of artificial neural networks, being connected to each other following the vascular graph.

6. The method of any of claims 3 to 5, wherein the training of the inverse artificial neural network based on the dataset comprises minimizing a first discrepancy between:

- the one or more measurements ($d$) of the vascular behavior of the patient, and
- a prediction of a vascular behavior simulation ($f(NN^{inv}(d))$) using the surrogate model and based on the predicted physiological parameters by the provided inverse artificial neural network.

7. The method of any of claims 3 to 6, wherein the training of the inverse artificial neural network based on the dataset comprises minimizing a second discrepancy ($\|NN^{inv}(d) - p\|$) between:

- the physiological parameters ($p$) reflecting the physiological features, and
- the determined physiological parameters ($NN^{inv}(d)$) by the provided inverse artificial neural network ($NN^{inv}$).

8. The method of any of claims 1 to 7, wherein the physiological parameters comprise at least one or more of:

- mechanical properties of the plurality of vessels, and/or
- geometrical properties of the plurality of vessels;

wherein, optionally, the one or more measurements comprises one or more pressure values and/or one or more flow rate values and/or one or more cross-sectional area values of the plurality of vessels.

9. The method of any of claims 1 to 8, wherein the predicting of the vascular behavior using the calibrated vascular model comprises:

- performing a simulation by the calibrated vascular model using the plurality of models, or
- predicting a simulation by the calibrated vascular model using the surrogate model.

10. The method of any of claims 1 to 9, further comprising, prior to the providing of a surrogate model, training the surrogate model, the method comprising:

- providing a training dataset, each entry in the training dataset comprises a value for the physiological parameters, and a value for a simulation by the vascular model using the plurality of models; and
- training the surrogate model using the training dataset.

11. The method of claim 10, further comprising, forming the provided training set; wherein the forming of the provided training set comprises:

- generating a plurality of set of values for the physiological parameters, each set being generated in a vicinity of a reference value; and
- performing a simulation by the vascular model using the plurality of models corresponding to the generated plurality of set of values for the physiological parameters.

12. A computer-implemented method of using the method of any of claims 1 to 11, the method of use comprising:

- obtaining the vascular model and the surrogate model;
- obtaining, from a user, the one or more measurements of the vascular behavior of the patient;
- calibrating the vascular model using the surrogate model and based on the one or more measurements;
- predicting the vascular behavior of the patient using the calibrated vascular model; and
- displaying, to a user, an avatar of a patient including a summary of the predicted vascular behavior;

wherein the method optionally further comprising:

- providing, by a user, an updated value for one or more of the plurality of the physiological parameters, thereby further calibrating the vascular model.

13. An inverse artificial neural network trained to predict physiological parameters of a patient given one or more measurements of a vascular behavior of the patient; optionally the inverse artificial neural network being trained according to any of claims 3 to 11.

14. A computer program comprising instructions for performing the method according to any one of claims 1 to 11 and/or the method of use according to claim 12.

15. A computer readable storage medium having recorded thereon the computer program of claim 14 and/or the trained inverse artificial neural network according to claim 13.

16. A system comprising a processor coupled to a memory and a graphical user interface, the memory having recorded thereon the computer program of claim 14.

providing a vascular model of a human circulatory system, the circulatory system comprising a plurality of vessels forming a vascular graph, the vascular model comprising a plurality of physiological parameters, the vascular model representing a general vascular behavior, the vascular model comprising a plurality of submodels each comprising a subset of the plurality of physiological parameters, each submodel of the plurality being respective to a vessel of a plurality of vessels forming a vascular graph; one or more measurements of the vascular behavior of the patient; and a surrogate model comprising an artificial neural networks the surrogate model being configured to predict a simulation of a vascular behavior from the physiological parameters — S10

calibrating the vascular model using the surrogate model and based on the one or more measurements — S20

predicting the vascular behavior of the patient using the calibrated vascular model — S30

FIG. 1

**FIG. 2**

EP 4 451 287 A1

FIG. 3

410

420

430

440

| Parametrize simulation configuration | → | Sampling for parameter space exploration | → | Generate simulation database | → | Train forward Neural Network |

470

| Acquire Patient measurements | → | Post process patient measurements | → | Train inverse Neural Network |

450

460

| Use the trained NNs to calibrate a new patient's parameters |

480

## FIG. 4

Input Parameters

Predicted Hemodynamics

## FIG. 5A

## FIG. 5B

Partial
Measurements

Reconstructed
Measurements

$f^0(t) = f^0(t_0) \ldots f^0(T)$

$g^0(t) = g^0(t_0) \ldots g^0(T)$

$f^M(t) = f^M(t_0) \ldots f^M(T)$

$g^M(t) = g^M(t_0) \ldots g^M(T)$

Measurement
reconstruction

$f^0(t) = f^0(t_0) \ldots f^0(T)$

$g^0(t) = g^0(t_0) \ldots g^0(T)$

$f^1(t) = f^1(t_0) \ldots f^1(T)$

$g^1(t) = g^1(t_0) \ldots g^1(T)$

$f^N(t) = f^N(t_0) \ldots f^N(T)$

$g^N(t) = g^N(t_0) \ldots g^N(T)$

## FIG. 6A

Reconstructed
Measurements

$f^0(t) = f^0(t_0) \ldots f^0(T)$

$g^0(t) = g^0(t_0) \ldots g^0(T)$

$f^1(t) = f^1(t_0) \ldots f^1(T)$

$g^1(t) = g^1(t_0) \ldots g^1(T)$

$f^N(t) = f^N(t_0) \ldots f^N(T)$

$g^N(t) = g^N(t_0) \ldots g^N(T)$

Artery 0

$p_0^0$
$p_1^0$
$p_2^0$

Artery 1

$p_0^1$
$p_1^1$
$p_2^1$

Artery N

$p_0^N$
$p_1^N$
$p_2^N$

Calibrated
Input
Parameters

$p_0^0$
$p_1^0$
$p_2^0$

$p_0^N$
$p_1^N$
$p_2^N$

## FIG. 6B

FIG. 7

FIG. 8A

FIG. 8D

FIG. 8B

FIG. 8C

Europäisches
Patentamt

European
Patent Office

Office européen
des brevets

# EUROPEAN SEARCH REPORT

Application Number

EP 23 30 5610

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Kissas Georgios ET AL: "Machine learning in cardiovascular flows modeling: Predicting arterial blood pressure from non-invasive 4D flow MRI data using physics-informed neural networks", arXiv.org e-Print archive, 17 September 2019 (2019-09-17), pages 1-30, XP093089277, arXiv:1905.04817 DOI: 10.48550/arXiv.1905.04817 Retrieved from the Internet: URL:https://arxiv.org/pdf/1905.04817.pdf [retrieved on 2023-10-06] * The whole document, in particular: Abstract; Pages 1, 2, 5, 10 – 13; Figures 2, 3. * | 1-16 | INV. G16H50/50 G06N3/045 |

-----

TECHNICAL FIELDS SEARCHED (IPC)

G16H
G06N

The present search report has been drawn up for all claims

| Place of search | Date of completion of the search | Examiner |
|---|---|---|
| Munich | 6 October 2023 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BOILEAU, ETIENNE ; PERUMAL NITHIARASU ; PABLO J. BLANCO ; LUCAS O. MÜLLER ; FREDRIK EIKELAND FOSSAN ; LEIF RUNE HELLEVIK ; WOUTER P. DONDERS ; WOUTER HUBERTS ; MARIE WILLEMET ; JORDI ALASTRUEY.** A Benchmark Study of Numerical Schemes for One-Dimensional Arterial Blood Flow Modelling. *International Journal for Numerical Methods in Biomedical Engineering,* 2015, vol. 31 (10), e02732
- **SHI, YUBING ; PATRICIA LAWFORD ; RODNEY HOSE.** Review of Zero-D and 1-D Models of Blood Flow in the Cardiovascular System. *BioMedical Engineering,* 26 April 2011, vol. 10 (1), 33
- Data Assimilation and Uncertainty Quantification in Cardiovascular Biomechanics. **LAL, RAJNESH.** PhD thesis. Université Montpellier, 2017
- **TEZZELE, MARCO ; FRANCESCO BALLARIN ; GIANLUIGI ROZZA.** *Combined Parameter and Model Reduction of Cardiovascular Problems by Means of Active Subspaces and POD-Galerkin Methods,* 2018, vol. 16, 185-207
- **KÖPPL, TOBIAS ; GABRIELE SANTIN ; BERNARD HAASDONK ; RAINER HELMIG.** Numerical Modelling of a Peripheral Arterial Stenosis Using Dimensionally Reduced Models and Kernel Methods. *International Journal for Numerical Methods in Biomedical Engineering,* August 2018, vol. 34 (8
- **FRESCA, STEFANIA ; ANDREA MANZONI.** Real-Time Simulation of Parameter-Dependent Fluid Flows through Deep Learning-Based Reduced Order Models. *Fluids,* 18 July 2021, vol. 6 (7), 259
- **SAITO, M. ; IKENAGA, Y. ; MATSUKAWA, M. ; WATANABE, Y. ; ASADA, T. ; LAGREE, P.Y.** One-dimensional model for propagation of a pressure wave in a model of the human arterial network: comparison of theoretical and experimental results. *Journal of Biomechanical Engineering,* 2011
- **LOMBARDI, D.** Inverse Problems in 1D Hemodynamics on Systemic Networks: A Sequential Approach: Unscented Kalman Filter Estimation of Network Parameters. *International Journal for Numerical Methods in Biomedical Engineering,* February 2014, vol. 30 (2), 160-79
- Gaussian process emulators for 1D vascular models. **ALESSANDRO MELIS.** PhD thesis. University of Sheffield, 2017
- **BLANCO et al.** Blood flow distribution in an anatomically detailed arterial network model: criteria and algorithms. *Biomech. Model. Mechanobiol.,* 2014, vol. 13, 1303-1330
- **BLANCO et al.** An anatomically detailed arterial network model for one dimensional computational hemodynamics. *IEEE Trans. Biomed. Eng.,* 2015, vol. 62, 736-53